# EUROPÄISCHE PATENTSCHRIFT

(11) **EP 3 947 086 B1**
(45) Veröffentlichungstag und Bekanntmachung des Hinweises auf die Patenterteilung: **03.07.2024**
(21) Anmeldenummer: 20714174.8
(22) Anmeldetag: 23.03.2020
(51) Int. Cl.: B60W 40/08, B60W 50/14

(54) **VERFAHREN UND EINRICHTUNG ZUR ÜBERWACHUNG EINES FAHRBETRIEBSBEDINGTEN GESUNDHEITSZUSTANDES VON INSASSEN EINES INSBESONDERE AUTONOMEN FAHRZEUGS**
METHOD AND DEVICE FOR MONITORING A STATE OF HEALTH, CONDITIONED BY A DRIVING OPERATION, OF OCCUPANTS OF AN, IN PARTICULAR, AUTONOMOUS VEHICLE
PROCÉDÉ ET DISPOSITIF DE SURVEILLANCE D'UN ÉTAT DE SANTÉ DÉPENDANT DU MODE DE CONDUITE D'OCCUPANTS D'UN VÉHICULE NOTAMMENT AUTONOME

(30) Priorität: 02.04.2019 DE 102019204691
(43) Veröffentlichungstag der Anmeldung: 09.02.2022
(73) Patentinhaber: carValoo GmbH, 45143 Essen (DE)
(72) Erfinder: RATH, Claudius, 45219 Essen (DE); SCHÖN, Nico, 45127 Essen (DE)
(74) Vertreter: Goebel, Sebastian
(86) Internationale Anmeldenummer: PCT/EP2020/057921
(87) Internationale Veröffentlichungsnummer: WO 2020/200862

(56) Entgegenhaltungen:
- DE-A1-102011 000 277
- DE-A1-102012 223 718
- DE-A1-102015 105 581
- US-A1- 2017 361 795
- US-A1- 2019 064 801

## Beschreibung

Die Erfindung betrifft ein Verfahren zur Ermittlung bzw. Überwachung eines fahrbetriebsbedingten Gesundheitszustandes von Insassen eines insbesondere autonom betriebenen Fahrzeugs, gemäß dem Oberbegriff des Anspruchs 1. Gegenstand der vorliegenden Erfindung sind auch ein Computerprogramm und eine Einrichtung, mittels derer das erfindungsgemäße Verfahren durchführbar ist.

### Stand der Technik

Bei Kraftfahrzeugen sind Sensorsysteme zur Überwachung von fahrbetriebsbedingten Informationen verfügbar, welche einem Fahrzeugbesitzer oder einer Werkstatt nur einen sehr begrenzten Zugang zu fahrdynamischen Sensordaten gewähren, um diese für Fahrzeugdiagnosezwecke oder für andere Zwecke zu verwenden.

Mit dem Aufkommen der Technologie autonomer Fahrzeuge bzw. Kraftfahrzeuge kann sich die Aufmerksamkeit des Fahrzeugführers auf alternative Aktivitäten wie z.B. Arbeit, Geselligkeit, Lesen, Schreiben, oder aufgabenbasierte Aktivitäten wie z. B. Organisation, Rechnungszahlungen, Online-Shopping, Computer- oder Onlinespiele, richten bzw. konzentrieren. Wenn sich das autonome Fahrzeug entlang einer vorgegebenen Fahrroute bewegt, kann sich beim Fahrer eine sogenannte "Kinetose", d.h. eine Reise- bzw. Bewegungskrankheit, einstellen. Die Ursache für diese Erkrankung liegt insbesondere darin, dass die Wahrnehmung der Fahrzeugbewegung seitens des Fahrers nicht der vestibulären Wahrnehmung des Fahrers mittels seines Gleichgewichtsorgans entspricht. Die medizinischen Symptome dieser Erkrankung können zudem durch Störungen des Gleichgewichtssinns einer Person entstehen.

Aus DE 10 2016 204 635 A1 gehen ein Verfahren und eine Vorrichtung zum Vermeiden oder Abmildern von Reiseübelkeit in einem autonomen Fahrzeug hervor, wobei ein für die Wahrscheinlichkeit des Auftretens von Reiseübelkeit bei einem Insassen des Fahrzeugs charakteristischer Kennwert abgeschätzt wird, wobei auf der Basis dieses Kennwertes wenigstens eine für die Abmilderung oder Vermeidung von Reiseübelkeit bei dem Insassen geeigneten Maßnahme festgelegt wird und wobei eine diese wenigstens eine Maßnahme betreffende Information an den Fahrer übermittelt wird. Als Maßnahmen kommen die Beendigung eines automatisierten Fahrmodus', die Vermeidung eines Wechselns in den automatisierten Fahrmodus, die Änderung oder Beendigung einer vom Fahrer aktuell durchgeführten Aktivität, die Änderung der Sitzposition des Fahrers oder die Änderung der Einstellung wenigstens einer Fensterabdeckung des Fahrzeugs in Betracht. Der Kennwert kann dabei auf der Basis von den Fahrer betreffenden Informationen durchgeführt werden, welche die Art einer vom Fahrer aktuell durchgeführten Aktivität, die Dauer einer zuvor erfolgten Durchführung der vom Fahrer aktuell durchgeführten Aktivität, die Dauer einer zuvor erfolgten Fahrt des Fahrzeugs in dem automatisierten Fahrmodus und/oder (bereits im Vorfeld bekannte) Gesundheitsdaten des Fahrers sein können. Der Kennwert kann auch auf der Basis von den Betriebszustand des Fahrzeugs betreffenden Daten durchgeführt werden, z.B. Beschleunigungsdaten des Fahrzeugs oder die Anregung des Fahrzeugs durch die Fahrbahnoberfläche.

Ferner geht aus US 2017/0313326 A1 ein sensorisches Stimulationssystem hervor, welches sensorische Stimulationsausgänge bereitstellt, die auf Fahrmanöver des autonomen Fahrzeugs ansprechen. Das Stimulationssystem umfasst eine Anzahl von Ausgabegeräten, mittels derer anhand von visuellen, akustischen oder taktilen Informationsquellen die vestibuläre Stimulation des Fahrzeugführers durch das jeweilige Fahrmanöver ermittelt wird. Dadurch kann den vestibulären Effekten, welche durch die Fahrzeugbewegung verursacht werden und zu einer genannten Kinetose führen können, entgegengewirkt werden. So kann das sensorische Stimulationssystem eine unterbewusste Lernatmosphäre innerhalb des Passagierinnenraums des autonomen Fahrzeugs bereitstellen, die eine Bewegungskrankheit verhindern und/oder ein präventives Feedback für den Fahrer bereitstellen kann. Das sensorische Stimulationssystem kann dabei zudem einen Passagier des Fahrzeugs mit einer visuellen Anzeige im Inneren des Fahrzeugs über ein bevorstehendes Fahrmanöver informieren. Ein genanntes Fahrmanöver kann eine Beschleunigung, Bremsung oder eine Richtungsänderung umfassen. Zu diesem Zweck umfasst das sensorische Stimulationssystem eine Anzahl von Sensoren, z.B. einen Beschleunigungsmesser oder einen Gyroskop-Sensor.

Die DE 10 2011 000 277 A1 offenbart ein Verfahren zur Ermittlung einer Unfallfolgenabschätzung für ein Notrufsystem eines Kraftfahrzeugs.

Die US 2017/361795 A1 offenbart ein Verfahren zur Unterstützung mindestens eines Insassen eines in einen Unfall verwickelten Fahrzeugs.

Die DE 10 2015 105 581 A1 betrifft ein System und ein Verfahren zur Überwachung des Gesundheitszustandes eines Fahrzeuginsassen.

Bislang existieren keine in das Fahrzeug integrierte oder nachrüstbare Systeme, welche die Insassen bzw. Passagiere (d.h. der Fahrer und/oder etwaige Mitfahrer) eines autonomen Fahrzeugs auf eine drohende Reisekrankheit (sog. "Kinetose" bzw. "Motion Sickness") hinweisen und auf der Grundlage eines erfassten Verhaltens der Insassen konkrete Maßnahmen vorschlagen, um eine Reisekrankheit wirksam zu verhindern. Es existieren zwar verschiedene Sensorsysteme zur Erfassung der Mimik, Gestik oder der Vitalfunktionen von Insassen eines Fahrzeugs. Diese werden jedoch eingesetzt, um primär den physischen Zustand des Fahrers zu erfassen, um dessen Fahreignung zu überwachen und ihm bei einer Nicht-Eignung entsprechende Hinweise zu geben, z.B. bei drohendem Sekundenschlaf oder wenn längere Zeit das Lenkrad nicht betätigt wird.

So bezieht sich die US 2017/0313326 A1 zwar auf einen ähnlichen Ansatz, bei dem in die Steuerung bzw. Regelung eines autonom fahrenden Fahrzeugs eingegriffen wird. Die Interaktion mit dem Fahrer beschränkt sich jedoch darauf, dem Fahrer eine optische, akustische oder haptische Orientierungshilfe für die Fahrstrategie seitens des autonom fahrenden Fahrzeugs zu geben.

### Offenbarung der Erfindung

Die Aufgabe der vorliegenden Erfindung besteht insbesondere darin, ein verbessertes Konzept für die Ermittlung einer möglichen Kinetose von Fahrzeuginsassen zu schaffen.

Die Aufgabe wird durch den Gegenstand der unabhängigen Patentansprüche gelöst. Weitere vorteilhafte Ausführungsformen sind der Gegenstand der abhängigen Patentansprüche.

Es wird ein insbesondere selbstlernendes Verfahren und eine Einrichtung zur Erfassung und Analyse von Sensordaten und zur Ermittlung einer möglichen Kinetose (Reisekrankheit) von Fahrzeuginsassen eines autonomen Fahrzeugs auf der Grundlage dieser Sensordaten vorgeschlagen. Anhand der Sensordaten werden z.B. Aufbaubewegungen der Fahrzeugkarosserie in einer Weise erkannt und bewertet, dass kritische Bewegungszustände des Fahrzeugs erkannt werden, die bei Fahrer oder Mitfahrern zu Symptomen der Reisekrankheit (auch "Bewegungskrankheit", "Motion Sickness" oder "Kinetose") führen können.

Dadurch können der Fahrer und etwaige Mitfahrer auf entsprechend kritische Bewegungszustände des Fahrzeugs aufmerksam gemacht werden, noch bevor diese zu spürbaren Krankheitssymptomen bei den Fahrzeuginsassen führen.

Zusätzlich können Vitalfunktionen des Fahrers und ggf. von Mitfahrern sensorisch oder per Benutzereingabe bzw. -feedback erfasst und bewertet werden, um das tatsächliche Wohlbefinden der Fahrzeuginsassen zu ermitteln. Der Fahrer und ggf. die Mitfahrer können dadurch frühzeitig auf kritische Bewegungszustände bzw. auf eine mögliche Beeinflussung ihres Gesundheitszustandes aufmerksam gemacht werden und darüber hinaus die Fahrstrategie ebenfalls frühzeitig angepasst werden und/oder weitere Maßnahmen ergriffen werden, welche das persönliche Wohlbefinden der Insassen erhalten oder noch verbessern können. Zu diesen Maßnahmen zählen z.B. eine gezielte Nahrungs- oder Flüssigkeitsaufnahme, eine Verhaltensanpassung oder die Anpassung der Raumtemperatur und/oder Luftfeuchte und/oder Frischluftzufuhr im Fahrzeuginnenraum. Als weitere Maßnahmen können auch technisch-optische Maßnahmen, z.B. ein künstlicher Horizont bei der Benutzung von mobilen digitalen Applikationen während der Fahrt, durchgeführt werden.

Im Gegensatz zum Stand der Technik ermöglichen das vorgeschlagene Verfahren und die Einrichtung nicht nur die Darstellung von Fahrzuständen des autonomen Fahrzeugs, sondern geben den Fahrzeuginsassen und/oder der Fahrzeugsteuerung konkrete Handlungsempfehlungen zur wirksamen Verhinderung einer Kinetose der Fahrzeuginsassen. Durch die zusätzliche sensorische Erfassung von Interaktionen oder Verhaltensweisen der Insassen, sowie insbesondere durch eine entsprechende Rückmeldungs- bzw. Feedbackschleife anhand der so erfassten Interaktionen, wird ein sehr wirksames, selbstlernendes Verfahren bzw. eine entsprechende Einrichtung ermöglicht.

Es können bei dem vorgeschlagenen Verfahren und der Einrichtung an dem Fahrzeug Sensoren zur genannten Datenerfassung vorgesehen sein, welche zumindest genannte Aufbaubewegungen bzw. Aufbaubeschleunigungen der Fahrzeugkarosserie und/oder Vitalfunktionen der Fahrzeuginsassen sensorisch erfassen. Solche Aufbaubewegungen betreffen einen bei einem Personenkraftwagen (Pkw) auf einem Fahrwerk üblicherweise angeordneten, den Fahrzeuginnenraum umfassenden Karosserieaufbau.

Es ist dazu anzumerken, dass ein derzeit im Pkw-Bau üblicher Aufbau meist aus punktgeschweißtem Blech, welches direkt mit dem Fahrwerk verbunden ist, gebildet ist. Der Aufbau überträgt somit sämtliche auf das Fahrwerk einwirkenden Kräfte z.B. an den Fahrzeuginnenraum.

Die von der Sensorik erfassten Daten werden insbesondere zur Auswertung der sensorisch erfassten Daten in Bezug auf eine möglicherweise drohende Kinetose von Fahrzeuginsassen hin bevorzugt drahtlos an einen externen Rechner, z.B. eine Cloud-Computing Plattform, übertragen. Der externe Rechner umfasst ein lernfähiges Auswertesystem, welches bevorzugt erfahrungsbasiert geeignete Maßnahmen zur wirksamen Verhinderung einer Kinetose vorschlägt. Die sensorisch erfassten Daten werden dabei in dem Fahrzeug fortwährend ausgelesen und zwischengespeichert und innerhalb eines vorgebbaren Zeitfensters erfasste Sensordaten auf das Vorliegen eines für die Ermittlung des fahrbetriebsbedingten Gesundheitszustandes des wenigstens einen Insassen relevantes Fahrereignis hin geprüft. Bei Überschreiten der Sensordaten eines empirisch vorgebbaren Schwellenwertes, bzw. bei entsprechend erkanntem Vorliegen eines für den Gesundheitszustand relevanten Ereignisses, werden die zwischengespeicherten Sensordaten des betreffenden Zeitfensters an den externen Rechner übermittelt, wobei bei nicht erkanntem Vorliegen eines relevanten Ereignisses in dem betreffenden Zeitfenster Nulldaten an den externen Rechner übermittelt werden.

Das lernfähige Auswertesystem kann dabei durch ein individuell auf wenigstens einen Fahrzeuginsassen abgestimmtes, selbstlernendes künstliches neuronales Netzwerk gebildet sein, wobei das selbstlernende künstliche neuronale Netzwerk Informationen über den Gesundheitszustand des wenigstens einen Fahrzeuginsassen einholt und diese Informationen mit den erfassten Sensordaten korreliert.

Es ist zudem anzumerken, dass die aufgezeichneten Sensordaten zur weiteren Verarbeitung bzw. Analyse der Sensordaten an einen externen Rechner übertragen werden, da aufgrund der Komplexität der Sensordaten sowie insbesondere in Anbetracht des dabei anfallenden Datenvolumens eine eingehende Datenauswertung nur mit einer relativ hohen Rechenleistung bzw. -kapazität möglich ist. Um das an den externen Rechner zu übertragende Datenvolumen zu reduzieren bzw. zu minimieren, ist in dem Fahrzeug eine Recheneinheit, z.B. ein Mikroprozessor oder ein Mikrocontroller, vorgesehen, mittels dessen die rohen Sensordaten so vorverarbeitet werden können, dass nicht sämtliche Rohdaten an den externen Rechner übertragen werden müssen.

Gemäß einem weiteren Aspekt kann vorgesehen sein, dass bei nicht erfolgtem Überschreiten des genannten, vorgegebenen Schwellenwertes ein Schlafmodus aktiviert wird und dass bei erfolgtem Überschreiten des vorgegebenen Schwellenwertes ein gegebenenfalls aktivierter Schlafmodus deaktiviert wird.

Die vorgeschlagene Einrichtung umfasst zu diesem Zweck eine Datenübertragungseinheit bzw. ein Kommunikationselement, welches zu möglichst vielen Zeitpunkten eine drahtlose Kommunikationsverbindung der Einrichtung mit einem genannten externen Rechner, z.B. als Mobilfunknetzmodul durch eine Funkverbindung über ein Mobilfunknetz, ermöglicht. Dadurch können die aufgezeichneten Sensordaten, unabhängig von der Fahrzeugposition, zeitweilig, regelmäßig oder fortwährend an den externen Rechner übertragen werden.

Die Sensorik umfasst gemäß einem weiteren Aspekt insbesondere Bewegungssensoren, welche dreiachsig die Beschleunigung sowie die Winkeländerung insbesondere in einem für das Entstehen von Kinetosen relevanten, niederfrequenten Bereich von bevorzugt ca. 0,1 bis 2,0 Hz erfassen.

Gemäß einem noch weiteren Aspekt können zusätzlich weitere Sensoren, wie z.B. Temperatur-, Luftfeuchtigkeitssensoren und/oder Gassensoren in dem Fahrzeug oder in einem mobilen Endgerät integriert angeordnet werden, mittels derer das Raumklima (Lufttemperatur, Luftfeuchtigkeit) und/oder die Luftqualität im Fahrzeuginnenraum erfasst bzw. ermittelt werden kann. Die Luftqualität kann den CO₂-Gehalt, flüchtige organische Verbindungen (sog. "Volatile Organic Compounds" = "VOC"), d.h. entsprechende oder anderweitig verursachte Geruchsbelastungen im Fahrzeuginnenraum betreffen. Diese Messgrößen sind für die Ermittlung des Gesundheitszustandes der Fahrzeuginsassen besonders geeignet, da die Luftqualität die generelle Belastbarkeit der Passagiere beeinflussen kann und z.B. bestimmte Gerüche mittelbarer oder unmittelbarer Auslöser z.B. von Übelkeit sein können.

Die genannten Sensoren können gemäß einem weiteren Aspekt entweder in das Fahrzeug bzw. in die Fahrzeugelektronik integriert sein oder z.B. mittels eines USB-Sticks oder eines 12V-Adapters (für den Zigarettenanzünder) in der Mittelkonsole des Fahrzeugs nachträglich installierbar ausgeführt sein. Alternativ kann die Sensorik mittels eines OBD-Dongles (OBD = Onboard-Diagnose), der mit einer Onboard-Elektronik des Fahrzeugs verbindbar ist, sich dort bereits vorhandene Sensorsignale und Informationen zunutze machen.

Gemäß einem noch weiteren Aspekt des vorgeschlagenen Verfahrens bzw. der Einrichtung werden die erfassten Sensorsignale durch eine Datenübertragungseinheit, z.B. eine Bluetooth-Schnittstelle, drahtlos an eine in dem Fahrzeug befindliche bzw. dort fest angeordnete Auswerte- und Visualisierungseinheit übertragen. Diese Einheit kann fest im Fahrzeug verbaut sein oder ein mobiles Endgerät darstellen, z.B. ein Smartphone, einen Tablet-PC oder eine Smartwatch.

Sollte das mobile Endgerät bereits über entsprechende Sensoren verfügen, können sowohl das Sensorsystem als auch die genannte Auswerte- und Visualisierungseinheit in einer Baueinheit bzw. in nur einem Gerät, z.B. in einem Smartphone oder in einer Smartwatch, integriert angeordnet sein. Das als Auswerte- und Visualisierungseinheit eingesetzte Endgerät besitzt dann gemäß einem zusätzlichen Aspekt des vorgeschlagenen Verfahrens bzw. der Einrichtung eine mobile Datenverbindung zum Internet, um die sensorisch erfassten Daten mit Erfahrungswerten in einer genannten Cloud-Computing Plattform zu vergleichen sowie mit Hilfe geeigneter Algorithmen dort zentral auswerten zu können.

Gemäß einem weiteren Aspekt ist das Auswertesystem als individuell auf den jeweiligen Fahrzeugführer und ggf. auf weitere Fahrzeuginsassen abgestimmtes, selbstlernendes System ausgelegt, wobei das selbstlernende System von dem System eindeutig bekannten Personen bzw. Fahrzeuginsassen regelmäßig Informationen über das medizinische bzw. körperliche Wohlbefinden dieser Personen einholt und diese Informationen mit den erfassten Sensordaten korreliert. Diese Informationen können, anstatt einer periodischen Erfassung, auch anlassbezogen oder durch die jeweilige Person initiiert eingeholt werden.

Gemäß einem noch weiteren Aspekt können dabei für bestimmte Fahrzeuginsassen individuelle bzw. personalisierte Empfindlichkeitsdaten bzw. entsprechende individuelle medizinische Dispositionsdaten berücksichtigt werden. Dabei kann nach erfolgter Identifikation eines einzelnen Fahrzeuginsassen, z.B. der (individuellen) Person XY, und nicht "des jeweiligen Passagiers hinten links", in einem bevorzugt längeren Beobachtungszeitraum Informationen über die individuelle Disposition dieser Person ermittelt und ggf. fortwährend verfeinert werden.

Diese Informationen können dann von einem entsprechenden, auf dem externen Rechnersystem gespeicherten (individuellen) Nutzerprofil dieser Person an das Fahrzeug (zurück) übertragen werden, und zwar bevorzugt dann, wenn diese Person das Fahrzeug betritt oder als Fahrzeugführer benutzt. Damit stehen diese Informationen in dem Fahrzeug für eine individualisierte Vorauswertung von während der Fahrt gewonnenen Daten zur Verfügung. Dies ist zum einen vorteilhaft, um die Qualität der an das externe Rechnersystem übertragenen Daten durch entsprechende Vorverarbeitung weiter zu verbessern, und zum anderen, um die Funktionsfähigkeit der vorgeschlagenen Einrichtung auch in Fahrsituationen zu gewährleisten, in denen eine mobile Datenverbindung mit dem externen Rechnersystem nur beschränkt oder gar nicht zur Verfügung steht, bei Fahrten in einem sog. "Funkloch".

Das jeweilige Endgerät, welches dann als Auswerte- und Visualisierungseinheit eingesetzt wird, besitzt gemäß einem weiteren Aspekt zudem eine entsprechende Benutzerschnittstelle, um dem Fahrer oder den Mitfahrern kritische Bewegungszustände mitzuteilen, und von diesen Personen eine Rückmeldung über ihr Wohlbefinden einzuholen. Die Benutzereingaben können dabei visuell über eine mobile Applikation (bzw. "App") eines Smartphones oder einer Smartwatch oder aber per Sprachsteuerung erfolgen.

Wird eine Smartwatch als Auswerte- und Visualisierungseinheit eingesetzt, können gemäß einem weiteren Aspekt zusätzlich die sensorisch erfassten Bewegungsdaten des Fahrzeugs zusätzlich mit von der Smartwatch bereitgestellten Sensordaten korreliert werden, welche aktuelle bzw. momentane Vitalfunktionen der jeweiligen Person (direkt) insbesondere im Fahrbetrieb des Fahrzeugs erfassen, z.B. den Puls, den Blutdruck, die Hautleitfähigkeit bzw. die Schweißproduktion oder andere mit einer Kinetose korrelierende Vitalfunktionen. Mittels einer entsprechend ausgerüsteten Smartwatch können zudem vorteilhaft auch weitere Vitalfunktionen eines betreffenden Fahrzeuginsassen, z.B. die Herzfrequenz und/oder ein vollständiges Elektrokardiogramm (EKG) sensorisch erfasst werden. Mittels an sich bekannter, tragbarer Messgeräte (sog. "wearable devices") kann zusätzlich die Atemfrequenz eines betreffenden Fahrzeuginsassen sensorisch erfasst werden.

Ein als Endgerät dienendes Smartphone, ein Tablet oder eine Smartwatch können gemäß einem weiteren Aspekt auch auf andere Informations- bzw. Datenquellen zugreifen und diese zusammenführen, z.B. smarte Textilien, Fitnessarmbänder, "In-car Monitoring Devices" (d.h. kamerabasierten bzw. optischen Systemen zur Erfassung von Vitalfunktionen im Fahrzeug, insbesondere Mimik, Gestik, Schluckbewegungen, Herzschlag, Blutdruck etc.) oder mobile medizinische Geräten mit drahtloser Datenschnittstelle (z.B. Sensoren zur Glukosemessung FGM = Flash Glucose Monitoring).

Die genannten Maßnahmen zur Erhaltung oder Verbesserung des persönlichen Wohlbefindens der Fahrzeuginsassen können gemäß einem weiteren Aspekt ebenfalls als selbstlernendes Maßnahmenvorschlagssystem ausgebildet sein. Ein solches System kann den Fahrzeuginsassen automatisch geeignete oder notwendige Maßnahmen zur Verhinderung einer Kinetose vorschlagen, z.B. die folgenden beispielhaften Maßnahmen bzw. Maßnahmenkategorien:
- Körperliche Maßnahmen, z.B. die Zufuhr von Flüssigkeit oder Nahrung;
- umgebungsbezogene Maßnahmen, z.B. die Anpassung der Fahrstrategie, der Innenraumtemperatur oder das Einlegen einer Fahrpause bzw. einer entsprechenden Ruhezeit, insbesondere außerhalb des Fahrzeugs;
- technisch bezogene Maßnahmen, z.B. die Darstellung eines künstlichen Horizonts an der Windschutzscheibe und/oder einer anderen Fensterscheibe oder an einem mobilen Endgerät mit einem Bildschirm und/oder durch angepasste Darstellung von Bildschirminhalten eines solchen Endgerätes parallel zu einem künstlichen Horizont, und zwar entsprechend der momentanen Fahrzeugbewegung bzw. -ausrichtung.

Die von dem mobilen Endgerät gelieferten Sensordaten können mittels einer Datenübertragungseinheit drahtlos an die in dem Fahrzeug angeordnete Auswerte-bzw. Verarbeitungs- und Visualisierungseinheit übertragen werden.

Darüber hinaus können die Analyseergebnisse des selbstlernenden Systems bzw. entsprechend vorgeschlagene Maßnahmen gemäß einem weiteren Aspekt an fahrzeugimmanente Regelfunktionen übermittelt werden, z.B. an das Fahrwerk und/oder eine (autonome) für das autonome Fahren des Fahrzeugs vorgesehene Fahrzeugregelung, und/oder an eine Sitzsteuerung oder eine Klimaanlage des Fahrzeugs.

Das vorgeschlagene Verfahren sieht gemäß einem noch weiteren Aspekt vor, dass an den von einer genannten Sensorik erfassten Bewegungsdaten des Fahrzeugs und ggf. der erfassten Vitalfunktionen der Fahrzeuginsassen eine zeitliche Fensterung dieser Daten in eine empirisch vorgebbare Fensterlänge durchgeführt wird. Innerhalb der so gebildeten Fenster wird eine Merkmalsextraktion zur Bereitstellung charakteristischer Merkmale in den Sensordaten anhand von zu erzeugenden Merkmalsvektoren sowie eine Klassifizierung der Sensordaten anhand der erzeugten Merkmalsvektoren durchgeführt.

Bei dem ersten Verfahrensschritt der Fensterung kann gemäß einem weiteren Aspekt zudem vorgesehen sein, dass mittels eines Beschleunigungssensors erfasste Beschleunigungsdaten sowie mittels eines Rotationssensors bzw. Gyrometers erfasste Rotationsdaten zunächst in kurze, leicht überlappende Fenster mit einer empirisch vorgebbaren, festen Zeitlänge unterteilt werden. Bevorzugt wird dabei eine Fensterlänge Δt von 0.5 - 20 s mit einer Überlappung von 1/8 der gesamten Fensterlänge verwendet. Es ist dabei anzumerken, dass die Fensterlänge von dem zu betrachtenden Frequenzspektrum der ggf. auftretenden Longitudinal-, Lateral-, und Vertikalbeschleunigungen des Fahrzeugs abhängig ist. So ergibt sich bei einer entsprechenden Schwingungsfrequenz f = 0.2 Hz eine Amplitudenlänge von 5 s und somit eine geeignete Fensterlänge von mindestens 10 s. Bei einer Frequenz f = 2 Hz ergibt sich jedoch eine Amplitudenlänge von 0.5 s und somit eine geeignete Fensterlänge von ebenfalls mindestens 2 s.

Bei dem zweiten Verfahrensschritt der Merkmalsextraktion kann gemäß einem weiteren Aspekt vorgesehen sein, dass für jedes so erzeugte Fenster verschiedene charakteristische Merkmale abgeleitet bzw. extrahiert werden. Aus den zeitabhängigen Rohdaten bzw. Rohzeitreihen extrahierte Merkmale können dabei entweder Zeitdomänenmerkmale wie z.B. Mittelwerte, Mediane, Standardabweichungen, mittlere Ableitungen, Standardabweichungsableitungen und Nulldurchgangsraten, oder Frequenzdomänenmerkmale wie z.B. Hochfrequenzanteile, Dominantfrequenzen oder Spektraldifferenzen sein. Mit solchen Merkmalen lässt sich jedes der erzeugten Fenster in jeder Achse der jeweiligen Rohzeitreihe durch einen hochdimensionalen Merkmalsvektor kennzeichnen.

In einem weiteren dritten Verfahrensschritt kann gemäß einem weiteren Aspekt vorgesehen sein, dass Übergangspunkte in der Abfolge genannter Merkmalsvektoren identifiziert werden, an denen abrupte Änderungen auftreten. Dabei kann ein multivarianter Algorithmus zur Wechselpunkterfassung anwendet werden. Diese Übergangspunkte ermöglichen es, ähnliche aufeinanderfolgende Fenster in unterschiedliche Arten der fahrdynamischen Belastung der Insassen des Fahrzeugs zu gruppieren.

Bei der Identifizierung von Übergangspunkten kann ein multivarianter Wechselpunkterfassungsalgorithmus angewendet werden, wobei ähnliche aufeinanderfolgende Fenster zu Segmenten gruppiert werden, wobei jedes Segment einer vorgebbaren Art der Fahrdynamik des Fahrzeugs entspricht.

In einem weiteren vierten Verfahrensschritt können die so ermittelten Merkmalsvektoren gemäß einem weiteren Aspekt z.B. unter Verwendung eines an sich bekannten "Random Forest"-Klassifikators, klassifiziert werden. Ein "Random Forest"-Klassifikator besteht bekanntermaßen aus mehreren unkorrelierten Entscheidungsbäumen, welche unter Verwendung eines Zufallsmechanismus' (sog. "Randomisierung") während eines zugrunde liegenden Lernprozesses gebildet werden. Für eine Klassifikation trifft jeder Entscheidungsbaum eine Entscheidung, wobei die Klasse mit den meisten Stimmen die endgültige Klassifikation festlegt.

Besonders rechenintensive Berechnungsschritte bei den genannten vier Verfahrensschritten können gemäß einem weiteren Aspekt auf dem genannten, außerhalb des Fahrzeugs angeordneten (externen) Rechner ausgeführt bzw. durchgeführt werden. Weniger rechenintensive Berechnungsschritte können dagegen bereits in dem Fahrzeug durchgeführt werden und nur entsprechend vorprozessierte Daten an den externen Rechner übertragen werden, um insbesondere das Datenvolumen der zu übertragenden Daten zu reduzieren bzw. zu minimieren.

Darüber hinaus können Parameter und Hyperparameter eines genannten Klassifikators anhand von Trainingsdaten gemäß einem weiteren Aspekt mittels an sich bekannter maschineller Lernalgorithmen ("machine learning algorithms") angelernt bzw. trainiert werden, wobei entsprechende Trainingsdaten bevorzugt für ein individuelles Fahrzeug oder einen Fahrzeugtyp fahrzeugspezifisch an einem jeweiligen Teststand erzeugt werden können. Zusätzlich werden die Trainingsdaten personenspezifisch erzeugt, und zwar z.B. anhand von Referenzdaten von Probanden mit ähnlichen Merkmalsausprägungen empirisch vorgebbarer Vitalfunktionen.

Alternativ oder zusätzlich können die Trainingsdaten, ebenfalls fahrzeugspezifisch und/oder personenspezifisch, bei Fahrten auf einer geeignet präparierten Teststrecke erzeugt werden.

Die fahrzeugspezifisch möglichen fahrdynamischen Zustände, welche die Aufbaubewegungen des Fahrzeugs bewirken, können zusätzlich mit personenspezifischen Merkmalsausprägungen (Vitalfunktionen) korreliert werden. So kann der Pulsschlag eines Insassen mit einer Aufbauanregung des Fahrzeugs mit einer bestimmten Frequenz- und/oder Amplitude zusammenhängen. Dabei liegt zu dem Erkenntnis zugrunde, dass eine gesundheitliche Wirkung, z.B. auf den Pulsschlag eines Insassen, mit einer Verzögerung (Latenz) von erst bis zu mehreren Minuten eintreten kann. Diese Latenz ist bei der Auswertung der erfassten Daten zu berücksichtigen, wobei ein geeigneter Wert der Latenz anhand von Trainingsdaten personenspezifisch erlernt werden kann.

Gemäß einem noch weiteren Aspekt des vorgeschlagenen Verfahrens bzw. der Einrichtung können die rohen Sensordaten fortwährend bzw. regelmäßig ausgelesen und zwischengespeichert werden. Die innerhalb eines empirisch vorgebbaren Zeitfensters erfassten Rohdaten werden dabei zunächst vorverarbeitet, wobei geprüft wird, ob ein ebenfalls empirisch vorgebbarer Schwellenwert einer sensorisch erfassten fahrdynamischen Größe oder einer sensorisch erfassten Vitalfunktion wenigstens eines der Fahrzeuginsassen überschritten wird und somit ein mögliches, den momentanen Gesundheitszustand des jeweiligen Fahrzeuginsassen beeinflussendes Fahrereignis vorliegt. Ein solches Fahrereignis kann durch die Fahrstrategie des autonomen Fahrzeugs, z.B. Beschleunigungen, Abbremsungen und/oder Lenkbewegungen, oder aber von der Fahrbahn, z.B. quer zur Fahrtrichtung verlaufende Bodenwellen oder besondere Straßenbeläge, hervorgerufen werden. Der Schwellenwert kann anhand eines empirischen, eigens parametrierten Datenmodells ermittelt werden, wobei das Datenmodell auf physikalischen Größen oder anderen Fahrzeugkenngrößen beruhen kann.

Dabei kann gemäß einem weiteren Aspekt vorgesehen sein, dass bei nicht erfolgtem Überschreiten des vorgegebenen Schwellenwertes ein Schlafmodus aktiviert wird und dass bei erfolgtem Überschreiten des vorgegebenen Schwellenwertes ein gegebenenfalls aktivierter Schlafmodus deaktiviert wird. Dabei können zudem während eines aktiven Schlafmodus' ausgelesene Sensordaten zunächst in einem Pufferspeicher zwischengespeichert werden und dass die in dem Pufferspeicher gespeicherten Sensordaten bei nicht erfolgtem Überschreiten des vorgegebenen Schwellenwertes dazu verwendet werden, um ein bezüglich eines relevanten Ereignisses vorheriges Zeitintervall der ursprünglich erfassten Sensordaten wiederherzustellen.

Das vorgeschlagene Verfahren und die Einrichtung ermöglichen es somit, dass die rechenintensive Datenanalyse nicht bereits in dem Fahrzeug erfolgen muss, sondern auf einem genannten externen Rechner durchgeführt werden kann. In dem Fahrzeug erfolgt lediglich eine Vorverarbeitung der erfassten Sensordaten, um für die nachfolgende, extern durchgeführte Datenanalyse irrelevante bzw. redundante Datenbestandteile herauszufiltern und somit den Datenverkehr über das meist vorliegende Mobilfunknetz zu minimieren.

Die rohen Sensordaten können vor dem Versenden an den externen Rechner noch durch die Einrichtung verschlüsselt werden, um eine ausreichende Datensicherheit für die ggf. personenbezogenen Benutzungs- und Vitalitätsdaten der Fahrzeuginsassen sicherzustellen.

Die Erfindung kann insbesondere bei einem autonom betriebenen Landfahrzeug, z.B. einem Personenkraftfahrzeug oder einem Nutzkraftfahrzeug, jedoch auch bei Wasserfahrzeugen, Unterwasserfahrzeugen, Luftfahrzeugen oder schienengebundenen Fahrzeugen, bei welchen es zu einer genannten Kinetose von Fahrzeuginsassen bzw. Passagieren kommen kann, entsprechend zum Einsatz kommen.

Das Computerprogramm ist eingerichtet, jeden Schritt des vorgeschlagenen Verfahrens durchzuführen, insbesondere wenn es auf einem externen Rechengerät oder einem an dem jeweiligen autonom betriebenen Fahrzeug angeordneten Rechengerät oder Steuergerät abläuft. Es ermöglicht die Implementierung des Verfahrens auch in einem bereits vorliegenden Steuergerät des Fahrzeugs, ohne an diesem bauliche Veränderungen vornehmen zu müssen. Hierzu ist der maschinenlesbare Datenträger vorgesehen, auf welchem das Computerprogramm gespeichert ist. Durch Aufspielen des Computerprogramms auf einen Mikroprozessor bzw. Mikrocontroller eines solchen Steuergerätes ist dieses eingerichtet, um das Verfahren auszuführen.

Weitere Vorteile und Ausgestaltungen der Erfindung ergeben sich aus der Beschreibung und den beiliegenden Zeichnungen.

Kurze Beschreibung der Zeichnungen
- Fig. 1: zeigt ein Ausführungsbeispiel der erfindungsgemäßen Einrichtung zur Überwachung des fahrbetriebsbedingten Gesundheitszustandes von Insassen eines autonomen Kraftfahrzeugs, und zwar schematisch anhand eines kombinierten Block-/Flussdiagramms.
- Fig. 2: zeigt ein Ausführungsbeispiel des erfindungsgemäßen Verfahrens zur Überwachung des fahrbetriebsbedingten Gesundheitszustandes von Insassen eines autonomen Kraftfahrzeugs, anhand eines kombinierten Block- /Flussdiagramms.
- Fig. 3: zeigt eine Übersicht einer beispielhaften Auswerteroutine zur Ermittlung des Gesundheitszustandes eines Insassen anhand eines Flussdiagramms.
- Fig. 4: zeigt eine noch detailliertere Darstellung der in Fig. 3 veranschaulichten Auswerteroutine anhand eines Flussdiagramms.
- Fig. 5: zeigt einen beispielhaften Auswertealgorithmus zur Implementierung der in Fig. 4 gezeigten Auswerteroutine anhand eines Flussdiagramms.

### Beschreibung von Ausführungsbeispielen

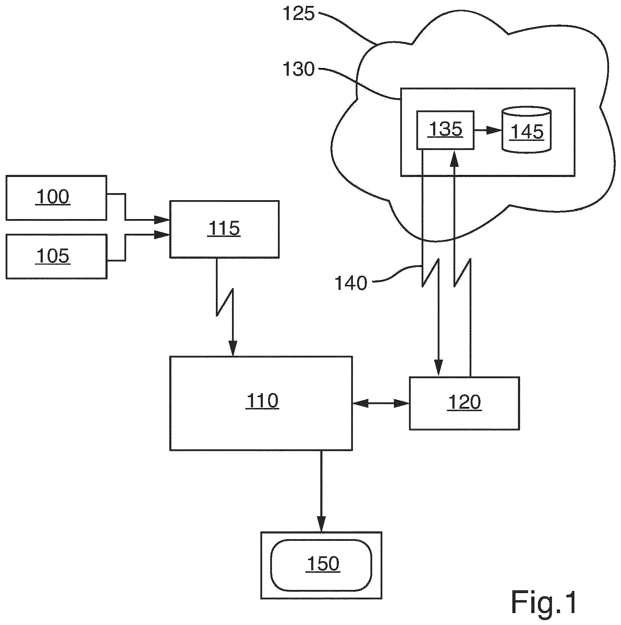

Die in Fig. 1 gezeigte Einrichtung umfasst in dem vorliegenden Ausführungsbeispiel wenigstens zwei Sensoren 100, 105 unterschiedlicher Kategorie. Der erste Sensor 100 der ersten Kategorie dient zur Erfassung von Daten bezüglich der Fahrdynamik des vorliegend angenommenen, autonom agierenden Kraftfahrzeugs. Dieser erste Sensor 100 dient in dem vorliegenden Ausführungsbeispiel zur Erfassung von eingangs genannten Aufbaubewegungen der Fahrzeugkarosserie. Der zweite Sensor 105 der zweiten Kategorie dient dagegen zur Erfassung von Vitalfunktionen der Fahrzeuginsassen bzw. Passagiere, insbesondere des Fahrzeugführers.

Die Einrichtung umfasst zusätzlich eine in bzw. an dem Fahrzeug angeordnete, Verarbeitungs- und Visualisierungseinheit 110 sowie eine erste Kommunikations- bzw. Datenübertragungseinheit 115 zur drahtgebundenen oder drahtlosen Übertragung der sensorisch erfassten Rohdaten an die Verarbeitungs-/Visualisierungseinheit 110, um diese zunächst im Fahrzeug vorzuverarbeiten. Die so vorverarbeiteten Daten werden mittels einer zweiten Kommunikations- bzw. Datenübertragungseinheit 120 drahtlos an ein außerhalb des Fahrzeug, in dem vorliegenden Beispiel an ein in einer Cloud-Computing Rechnerplattform 125 angeordnetes Auswertesystem 130 übertragen, welches die Daten mittels lernfähiger, künstlicher Intelligenz (KI) 135 auswertet und auf dieser Grundlage den Insassen des Fahrzeugs erfahrungsbasiert geeignete Maßnahmen zur Verhinderung eines Unwohlseins oder einer Kinetose der Insassen gemäß der in der Pfeilrichtung 140 an das Fahrzeug zurück übertragenen Daten vorschlägt.

Die Verarbeitungs- und Visualisierungseinheit 110 ist in dem vorliegenden Ausführungsbeispiel mit einem an der Mittelkonsole des Fahrzeugs angeordneten Bildschirm 150 verbunden, welcher die von dem Auswertesystem 130 vorgeschlagenen Maßnahmen an die Fahrzeuginsassen kommuniziert. Ein solcher Bildschirm kann auch in eine der Glasscheiben des Fahrzeugs, z.B. in die Windschutzscheibe oder in eine Seitenscheibe, integriert angeordnet sein. Die Kommunikation mit den Fahrzeuginsassen kann auch in anderer audio-visueller Art erfolgen, ggf. auch interaktiv mit dem jeweiligen Fahrzeuginsassen mittels einer in der Verarbeitungs- und Visualisierungseinheit 110 eigens angeordneten Kl, um die für diese Person geeignetste Maßnahme herauszufinden.

Die drahtlose Übertragung der bereits vorverarbeiteten Daten mittels der zweiten Datenübertragungseinheit 120 erfolgt z.B. mittels eines Mobilfunknetzes wie z.B. des Universal Mobile Telecommunications Systems (UMTS). Diese Übertragung kann aber auch mittels alternativer Übertragungstechniken erfolgen, z.B. mittels eines "Wide Area Networks", welches ein Rechnernetz darstellt, welches sich im Unterschied zu einem LAN oder MAN über einen sehr großen geografischen Bereich erstreckt.

Der erste Sensor 100 ist in dem vorliegenden Ausführungsbeispiel durch einen Bewegungssensor gebildet, welcher sowohl dreiachsig die Fahrzeugbeschleunigung mittels eines Beschleunigungssensors als auch dreiachsig Winkeländerungen bezüglich der Fahrtrichtung des Fahrzeugs mittels eines Drehbewegungen oder Lageänderungen des Fahrzeugs erfassenden Gyrosensors, und zwar jeweils in einem relativ niederfrequenten Abtastbereich erfasst. Wie bereits erwähnt, ist die Fensterlänge von dem zu betrachtenden Frequenzspektrum der ggf. auftretenden Longitudinal-, Lateral-, und Vertikalbeschleunigungen des Fahrzeugs abhängig. Zusätzlich können dabei auch Rotationsbeschleunigungen, z.B. Nicken, Rollen und/oder Gieren des Fahrzeugs, sowie deren zeitliche Änderungen (Rucke), erfasst und bewertet werden. Dabei gilt die Bedingung Abtastfrequenz > 2 * Signalfrequenz. Damit ergeben sich typischerweise Frequenzen von f = 0,2 Hz bis f = 2 Hz. Anhand der so sensorisch erfassten Daten werden vorliegend Longitudinal-, Lateral-, und Vertikalbeschleunigungen des Fahrzeugs im Amplitudenbereich von bevorzugt bis ca. 1 g in dem genannten Frequenzbereich erkannt.

Es ist hierbei anzumerken, dass als Sensor 100 der genannten ersten Kategorie zusätzlich auch ein Temperatur- und/oder Luftfeuchtigkeitssensor oder dergleichen vorgesehen sein kann, anhand dessen die klimatischen Verhältnisse im Innenraum des Fahrzeugs ermittelt werden können. Solche zusätzlichen Sensoren können dabei, anstatt an dem Fahrzeug fest installiert zu sein, auch z.B. in einem USB-Stick oder an einem 12V-Adapter für den Zigarettenanzünder des Fahrzeugs angeordnet sein. Ein solcher USB-Stick kann zudem mittels einer in der Mittelkonsole des Fahrzeugs vorgesehenen Datenschnittstelle angeschlossen werden. Dadurch ist auch die Stromversorgung des USB-Sticks während der Fahrt gesichert.

Die genannte Sensorik 100, 105 kann insgesamt oder teilweise auch in einer Onboard-Elektronik des Fahrzeugs integriert sein.

Die erfassten Sensorsignale werden mittels der genannten ersten Datenübertragungseinheit 115, in dem vorliegenden Ausführungsbeispiel eine "Bluetooth"-Schnittstelle, drahtlos an die Verarbeitungs-/Visualisierungseinheit 110 übertragen. Diese Übertragung der Sensor-Rohdaten kann aber auch kabelgebunden erfolgen, da sie nur den Fahrzeuginnenraum betrifft.

Die Verarbeitungs-/Visualisierungseinheit 110 kann in dem Fahrzeug fest angeordnet sein und sich dabei eine in dem Fahrzeug bereits vorhandene Benutzerschnittstelle bzw. Mensch-Maschine-Schnittstelle zunutze machen. Eine solche Schnittstelle kann z.B. in das Armaturenbrett integriert sein oder in einem üblicherweise an der Mittelkonsole des Fahrzeugs angeordnetes Multimediasystem (z.B. einschließlich eines Radios und/oder eines Navigationssystems) integriert sein.

Alternativ kann die Verarbeitungs-/Visualisierungseinheit 110 auch durch ein mobiles Endgerät realisiert sein, z.B. ein Smartphone oder eine Smartwatch. Sollte das mobile Endgerät über entsprechende Sensoren verfügen, kann zusätzlich zur Verarbeitungs-/Visualisierungseinheit 110 auch die genannte Sensorik 100, 105 in das Smartphone oder die Smartwatch physisch integriert sein.

Ein in dieser Weise eingesetztes mobiles Endgerät weist bevorzugt eine Recheneinheit mit einem Datenspeicher, einen berührungsempfindlichen Bildschirm (Touchscreen) und/oder eine Sprachsteuerung auf. Zusätzlich weist das Endgerät eine genannte Datenübertragungseinheit zur Herstellung einer mobilen Datenverbindung mit dem Internet auf. Die Verbindung mit dem Internet erfolgt dabei bevorzugt mittels eines Datengateways, auf dem ein Betriebssystem sowie spezielle Anwendungsprogramme (z.B. "Apps") ausgeführt werden können.

Mittels der Anwendungsprogramme werden die Sensorsignale sowie zusätzlich eine Rückmeldung bzw. ein Nutzerfeedback seitens der Fahrzeuginsassen verarbeitet und insbesondere zusammengeführt. Anschließend werden die so vorverarbeiteten Daten über das genannte Datengateway an ein genanntes zentrales Analyse-/Auswertesystem 130 z.B. einer Cloud-Computing Plattform 125 übermittelt.

Darüber hinaus können mittels eines genannten mobilen Anwendungsprogramms die an das mobile Endgerät zurück übertragenen Ergebnisse der extern durchgeführten Datenanalyse bzw. -auswertung in Form einer Empfehlung visuell oder sprachlich dem Nutzer zur Verfügung gestellt werden.

Das externe, vorliegend in einer zentralen Cloud-Plattform bereitgestellte Analyse- bzw. Auswertesystem 130 tauscht seine Daten mit dem Fahrzeug bidirektional über ein genanntes Datengateway drahtlos aus. Das Analyse-/Auswertesystem weist eine Datenbank 145 auf, in der die zu verarbeitenden Daten, d.h. die vorverarbeiteten Sensordaten, die vorliegenden Daten zum Nutzerfeedback sowie die als Ergebnis der Analyse bzw. Auswertung empfohlenen Maßnahmen, aufbereitet, strukturiert und gespeichert werden.

Die in der Datenbank enthaltenen Daten werden anhand eines Datenmodells miteinander korreliert, verglichen und entsprechend analysiert. Die Datenbank umfasst zusätzlich personenindividuelle Erfahrungswerte und generiert jeweils neue Erfahrungswerte. Auf der Grundlage der genannten Daten sowie der Erfahrungswerte schlägt das Analyse-/Auswertesystem spezifische Maßnahmen vor, durch die die Fahrzeuginsassen ihr Wohlbefinden verbessern können bzw. eine Kinetose (Reisekrankheit) wirksam zu verhindern.

Das Analyse-/Auswertesystem ist in dem vorliegenden Ausführungsbeispiel als, auf den jeweiligen Fahrzeuginsassen individuell abgestimmt, selbstlernendes System ausgelegt. Zu diesem Zweck fordert das System von den jeweils betroffenen Personen regelmäßig eine Nutzerinteraktion bzw. Rückmeldung (Feedback) zu ihrem derzeitigen Wohlbefinden an, um diese zusätzlichen Informationen mit den erfassten Sensordaten zu korrelieren.

Die Analyseergebnisse insbesondere zu möglichen kritischen Bewegungszuständen des Fahrzeugs werden dem bzw. den jeweiligen Fahrzeuginsassen mittels eines mobilen Endgerätes oder einer im Fahrzeug fest angeordneten Visualisierungseinheit mitgeteilt. Auch die genannten Nutzerinteraktionen können entweder mittels des mobilen Endgerätes oder mittels einer im Fahrzeug fest angeordneten Verarbeitungs-/Visualisierungseinheit erfasst werden.

Anhand von dem bzw. den Fahrzeuginsassen etwa mitgeteilten, für das Wohlbefinden möglicherweise kritischen Bewegungszuständen des Fahrzeugs wird hierzu eine Rückmeldung des jeweils betroffenen Insassen über ihr/sein Wohlbefinden eingeholt. Diese Rückmeldung kann visuell über ein in dem jeweiligen Endgerät vorliegendes Anwendungsprogramm und dabei ggf. auch per Spracheingabe bzw. Spracherkennung sowie ggf. zusätzlich per Gestenerkennung erfolgen.

Wird als mobiles Endgerät eine Smartwatch eingesetzt, können die sensorisch erfassten Bewegungsdaten zusätzlich mit Vitalfunktionen des jeweiligen Uhrenträgers betreffenden Sensordaten der Smartwatch korreliert werden. Als solche Vitalfunktionen kommen z.B. der Puls, der Blutdruck, der Insulinspiegel, die Hautleitfähigkeit bzw. die Schweißproduktion oder ähnliche physiologische Messgrößen in Betracht.

Die von dem Analyse-/Auswertesystem vorgeschlagenen Maßnahmen bzw. Handlungsempfehlungen können visuell oder sprachlich bzw. akustisch dem bzw. den Fahrzeuginsassen gegenüber ausgegeben werden. Als Maßnahmen kommen z.B. die folgenden Handlungen bzw. Handlungskategorien in Betracht:
- Körperliche Maßnahmen, z.B. die zeitnahe Zufuhr von Flüssigkeit und/oder Nahrung, um das Wohlbefinden zu verbessern;
- Bezüglich der näheren Umgebung der Fahrzeuginsassen bezogene Maßnahmen, z.B. eine geeignete Anpassung der Fahrstrategie des autonomen Fahrzeugs, eine geeignete Anpassung der Innenraumtemperatur des Fahrzeugs oder das Einlegen einer Fahrpause, insbesondere außerhalb des Fahrzeugs zu Erholungszwecken des jeweiligen Insassen;
- technisch bezogene Maßnahmen, z.B. die Darstellung eines künstlichen Horizonts an der Windschutzscheibe und/oder einer anderen Fensterscheibe oder an einem mobilen Endgerät mit einem Bildschirm. Alternativ oder zusätzlich können Bildschirminhalte eines Endgerätes entsprechend an einen solchen künstlichen Horizont angepasst werden, und zwar bevorzugt in Bezug auf die momentane Fahrzeugbewegung bzw. Fahrzeugausrichtung.

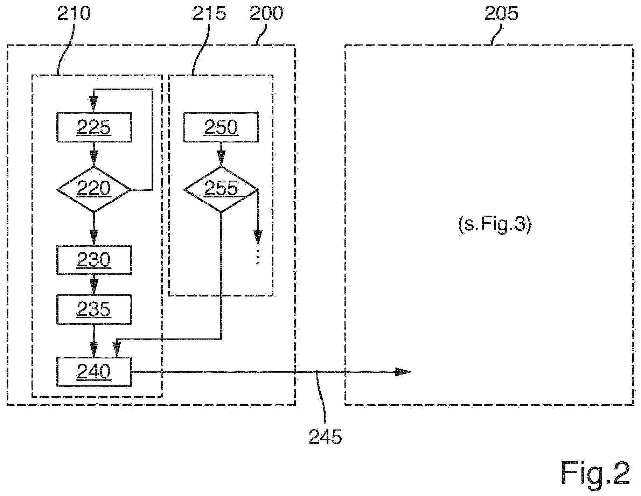

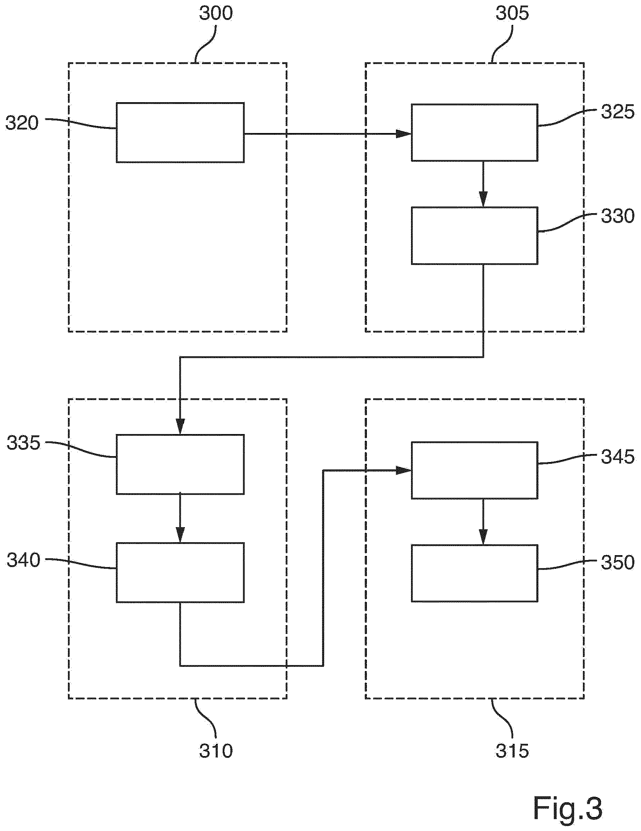

Das in Fig. 2 gezeigte Ausführungsbeispiel des erfindungsgemäßen Verfahrens zur Überwachung des Gesundheitszustandes von Insassen eines hier betroffenen Fahrzeugs setzt sich aus einem in dem Fahrzeug durchgeführten Vorverarbeitungsabschnitt und einem bevorzugt außerhalb des Fahrzeugs durchgeführten Analyse- bzw. Auswerteabschnitt zusammen, welche durch gestrichelte Linien 200, 205 voneinander abgegrenzt sind. Der Analyse- bzw. Auswerteabschnitt wird nachfolgend anhand von Fig. 3 beschrieben.

Die im Vorverarbeitungsabschnitt 200 vorgesehenen Prozessschritte werden in dem vorliegenden Ausführungsbeispiel in einem an dem Fahrzeug angeordneten Mikrocontroller durchgeführt. Dabei sind zwei Prozessbereiche vorgesehen, und zwar zum einen eine Weck/Schlaf-Routine 210 und eine Vorverarbeitungsroutine 215 zur Vorverarbeitung der zunächst noch rohen Sensordaten.

Bei der Weck/Schlaf-Routine 210 werden während des Fahrbetriebs des Fahrzeugs kontinuierlich Rohdaten von dem Beschleunigungssensor 100 ausgelesen. Die so erfassten Rohdaten werden in empirisch vorgebbare Zeitfenster Δt unterteilt und in jedem dieser Zeitfenster eine statistische Auswertung durchgeführt, auf deren Basis eine Ereigniserkennung 220 erfolgt. Auf der Grundlage der Ereigniserkennung 220 wird mittels einer Entscheidungslogik 225 erkannt, ob ein für den hier betroffenen Gesundheitszustand von Fahrzeuginsassen relevantes, fahrdynamisches Ereignis vorliegt, aufgrund dessen die Weck/Schlaf-Routine aus einem Schlafmodus in einen aktiven Modus übergeführt wird.

Der Betrieb der Weck/Schlafroutine 210 beruht auf den folgenden Prozessbedingungen:
- Der Schlafmodus wird immer dann aktiviert 220, wenn für einen empirisch vorgebbaren Zeitraum Δt_{idle} kein genanntes Ereignis erkannt worden ist. Dabei kann ein empirisch vorgebbarer Schwellenwert 225 zugrunde gelegt werden, wobei nur bei dessen Überschreiten ein solches fahrbetriebsbedingtes Ereignis erkannt wird.
- Während des Schlafmodus' ist ausschließlich der wenigstens eine Sensor 100 der ersten Kategorie, z.B. ein Beschleunigungssensor, aktiv.
- Der Beschleunigungssensor 100 versorgt den Mikrocontroller fortwährend mit Messdaten.
- Sobald ein genanntes, relevantes Ereignis innerhalb eines ebenfalls empirisch vorgebbaren Zeitfensters Δt erkannt wird, wird der Schlafmodus deaktiviert 230 und weitere Prozessroutinen 235 gemäß der nachfolgenden Vorverarbeitungsroutine gestartet ("aktiver Modus").

Bei der Vorverarbeitungsroutine 215 werden definierte Zeitfenster Δt auf den sensorisch erfassten Signalverläufen abgebildet 250, welche zeitlich überlappend sind. Dabei werden die Signalverläufe der Rohdaten mit einem empirisch vorgebbaren Schwellenwert verglichen 255. Bei Überschreitung des Schwellenwertes werden die in dem jeweils betrachteten Zeitfenster Δt enthaltenen, vorliegend aus den sechs Messgrößen aₓ, a_{y} und a_{z} sowie gₓ, g_{y} und g_{z} gebildete Daten vollständig, und einen Zeitstempel umfassend, in einem nicht-flüchtigen Datenspeicher, z.B. einem Flash-Speicher, zwischengespeichert 240. Die in dem Flash-Speicher gespeicherten Daten werden regelmäßig an den externen Rechner zur dortigen Weiterverarbeitung übertragen 245.

Wurde bei der Vorverarbeitung kein relevantes Ereignis erkannt, dann werden "Nulldaten" erzeugt, diese Nulldaten in dem Flash-Speicher abgelegt 240 und anschließend ebenfalls an den externen Rechner übermittelt 245.

Die in Fig. 3 gezeigte und nachfolgend beschriebene Auswerteroutine zur Beurteilung bzw. Ermittlung des Gesundheitszustandes eines der Fahrzeuginsassen wird in dem vorliegenden Ausführungsbeispiel in einer Cloud-Computing Rechnerplattform ausgeführt. Diese Routine setzt sich in dem vorliegenden Ausführungsbeispiel aus vier jeweils durch gestrichelte Linien abgegrenzten Prozessbereichen zusammen, nämlich eine Fensterung 300, Merkmalsextraktion 305, Segmentierung 310 und eine Klassifizierung 315 die jeweils erfassten Sensordaten betreffend.

Gemäß dem ersten Prozessabschnitt 300 werden zunächst in dem vorliegenden Ausführungsbeispiel von dem Sensor 100 der ersten Kategorie erfasste Beschleunigungs- und Gyrationszeitreihendaten in kurze, leicht überlappende Fenster mit einer empirisch vorgebbaren, festen Zeitlänge unterteilt 320. Bevorzugt wird dabei eine Fensterlänge von 2 - 10 s mit einer Überlappung von 1/8 der gesamten Fensterlänge verwendet. Für jedes so erzeugte Fenster werden verschiedene charakteristische Merkmale abgeleitet bzw. extrahiert 325. Solche extrahierten Merkmale können Zeitdomänenmerkmale, z.B. ein Mittelwert, ein Median, eine Standardabweichung, eine mittlere Ableitung, eine Standardabweichungsableitung oder eine Nulldurchgangsrate sein.

Alternativ oder zusätzlich können solche extrahierten Merkmale Frequenzdomänenmerkmale, z.B. ein Hochfrequenzanteil, eine Dominantfrequenz oder eine Spektraldifferenz sein. Mit diesen extrahierten Merkmalen wird dann jedes der erzeugten Fenster in jeder Achse der Rohzeitreihe durch einen hochdimensionalen Merkmalsvektor gekennzeichnet 330.

Wenn die Zeitreihendaten in der genannten Weise segmentiert sind, werden die Merkmalsvektoren von Fenstern innerhalb jedes Segments aggregiert 345, was einen aggregierten Merkmalsvektor pro Segment ergibt. Diese Merkmalsvektoren werden schließlich zur Klassifizierung 350 von möglichen, fahrbetriebsbedingten Gesundheitszuständen eines Fahrzeuginsassen unter Verwendung eines sogenannten "Random Forest"-Klassifizierers verwendet.

Von dem Sensor 105 der zweiten Kategorie erfasste Vitalfunktionen eines betrachteten Fahrzeuginsassen, und zwar vorliegend des Fahrzeugführers, können in der beschriebenen Weise entsprechend ausgewertet werden. Alternativ oder zusätzlich können die von dem zweiten Sensor 105 gelieferten Daten mit den von dem ersten Sensor 100 gelieferten Daten korreliert werden, wodurch sich die gesundheitliche Aussagekraft der Analyse- bzw. Auswerteergebnisse erheblich verbessern lässt. Die Bedeutung dieser Korrelationen für das zu ermittelnde Gesundheitsbild eines Fahrzeuginsassen können dabei auch mittels der genannten Kl 135 antrainiert bzw. gelernt werden.

Zur Erkennung von Änderungen des Mittelwerts oder der Varianz von in dem Zeitintervall Δt enthaltenen Sensordaten wird in dem vorliegenden Ausführungsbeispiel zunächst eine Sequenz von n Zufallsvariablen {*X*1, ... , *Xn*} vorgegeben. Dabei wird angenommen, dass bereits eine geeignete Klassifizierung für die möglichen bzw. verschiedenen Änderungen vorliegt. Die Zufallsvariablen dienen sozusagen als Platzhalter für die vorliegend auszuwertenden Sensordaten, d.h. bevorzugt von Beschleunigungsdaten und gyroskopischen Daten. Aus einer Anzahl möglicher Gesundheitsstatistiken wird nun eine bestimmte Statistik ausgewählt, welche für eine vorgegebene Änderungsklasse eine geeignete oder die optimale Gesundheitsstatistik darstellt. Bei den genannten Statistiken kann es sich z.B. um die an sich bekannte Methode des "Generalized Likelihood Ratio" (GLR) Tests, um eine anhand von Testpersonen ermittelte Gesundheitsstatistik in Bezug auf die Kinetose oder um die ebenfalls an sich bekannte Methode der sogenannten "Fishers Exact Test" (FET) Statistik handeln, welche üblicherweise zur Erkennung von Parameteränderungen in einer Bernoulli-Sequenz eingesetzt wird, handeln.

Danach wird festgelegt, wie groß die durchschnittliche Anzahl von erkannten Änderungen sein muss, damit überhaupt eine Fehlererkennung erfolgt bzw. ein Fehler erkannt wird. Dabei werden Werte für eine Falschalarmrate und eine durchschnittliche Lauflänge ("Average Run Length" = ARL0) empirisch vorgegeben. Eine zwei Stichproben ("two-sample") umfassende Teststatistik *Dk,n* wird an jedem möglichen Splitpunkt ("split point") *Xk* des zu untersuchenden Datensatzes ausgewertet bzw. bewertet. Dabei wird mittels einer genannten Teststatistik und der ARL0 ein Schwellenwert *hn* berechnet. Dabei wird auf der Grundlage des Schwellenwertes *hn,* und zwar bei Erfülltsein der Bedingung *max Dk,n* > *hn,* ein Zeitpunkt *T,* zu dem eine jeweils erkannte Änderung stattgefunden hat, gemäß der Beziehung *T* = arg(*k*) max *Dk,n* berechnet.

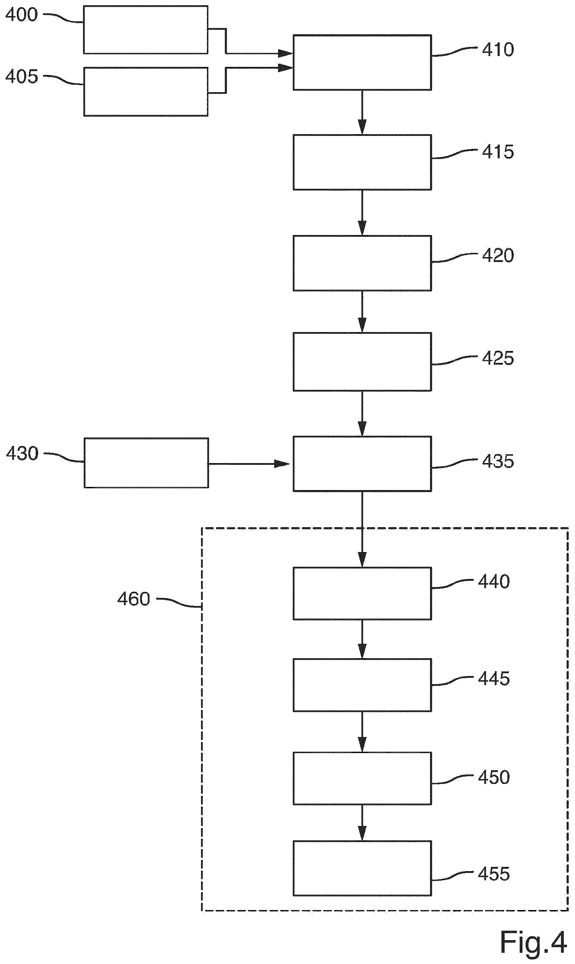

Wie aus der Fig. 4 zu ersehen, werden die von dem ersten Sensor 100 gelieferten Beschleunigungs- 400 und Winkeldaten 405 wie folgt ausgewertet. Diese Daten 300, 305 werden zunächst in der Zeitdomäne analysiert 410, um Zeitbereiche mit anomalen Daten Beschleunigungs- bzw. Rotationsdaten zu erkennen und zu selektieren 415. Die in den so selektierten Zeitbereichen verfügbaren Daten werden in die Frequenzdomäne umgewandelt 420.

Die so selektierten Messsignale werden anhand von für vorliegend relevante Fahrzeugereignisse empirisch vorgebbaren Wavelets 430 analysiert 435, d.h. in dem vorliegenden Ausführungsbeispiel durch Vergleich von entsprechend gegenüberstehenden Signalverläufen. Es ist anzumerken, dass die so vorgegebenen Wavelets im Vorfeld z.B. an einem Fahrzeug-Teststand ermittelt werden können, wobei bevorzugt ein Lernprozess durchgeführt werden kann. Anhand des Lernprozesses kann der charakteristische Signalverlauf für ein bestimmtes, den Gesundheitszustand der Fahrzeuginsassen beeinflussendes Fahrereignis optimiert werden.

Anhand der Wavelets können die Messsignale ggf. weiter segmentiert 440 und danach entsprechend von Wavelet-Gruppen eingruppiert werden 445. Die sich dabei ergebende Gruppierung der Messsignale ermöglicht die Bildung 450 von Clustern, anhand derer danach die Klassifizierung der Messdaten in Bezug auf ein vorliegend erkanntes Ereignis durchgeführt wird 455.

Die Ergebnisse der Klassifizierung 455 ermöglichen präzise Aussagen über den jeweiligen, mechanischen Fahrbetriebszustand des Fahrzeugs, insbesondere von Änderungen des Fahrbetriebszustandes aufgrund eines besonderen Fahrverhaltens des autonomen Fahrzeugs. Mögliche Fahrbetriebszustände werden dabei bevorzugt mit genannten Wavelets 430 in Beziehung gesetzt bzw. mit diesen korreliert. Solche Korrelationen können zudem anhand von realen Messdaten gelernt bzw. optimiert werden. Dabei können die durch die gestrichelte Linie 460 eingegrenzten Verfahrensschritte entsprechend wiederholt bzw. rekursiv ausgeführt werden.

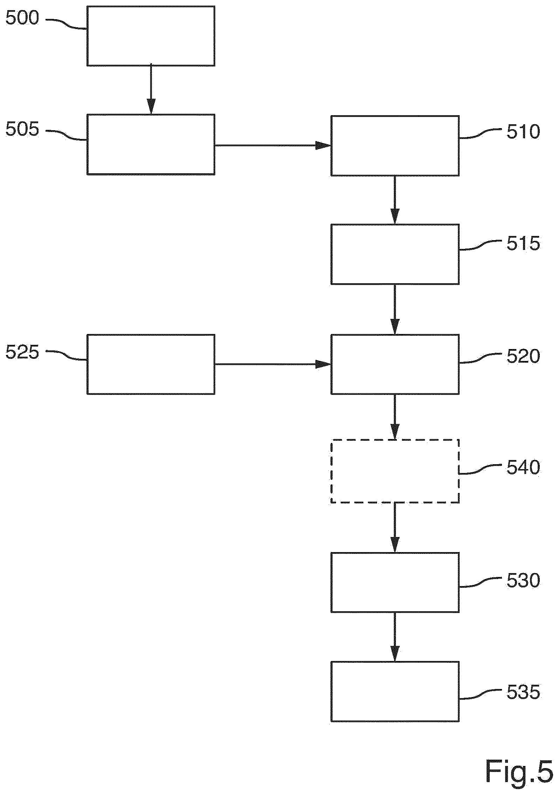

Die Fig. 5 zeigt einen Auswertealgorithmus zur Implementierung des anhand von Fig. 4 beschriebenen Auswerteverfahrens zur Analyse der an dem Fahrzeug erfassten Sensordaten (Sensor 100 in Fig. 1) gemäß der genannten ersten Kategorie. Dabei werden in dem vorliegenden Ausführungsbeispiel die erfassten Beschleunigungs- und Gyrationsdaten zunächst in Zeitfenster mit einer vorgegebenen Zeitlänge von 2 - 10 s und mit einer Überlappung von 1/8 der gesamten Fensterlänge unterteilt 500. Für diese Fenster werden vorliegend als charakteristische Zeitdomänenmerkmale ein Mittelwert, eine Standardabweichung sowie eine mittlere zeitliche Ableitung extrahiert 505. Zusätzlich werden aus den Daten in dem vorliegenden Ausführungsbeispiel ein Hochfrequenzanteil sowie eine Spektraldifferenz extrahiert 510. Mit diesen extrahierten 505, 510 Merkmalen wird jedes der erzeugten Fenster durch einen genannten, hochdimensionalen Merkmalsvektor gekennzeichnet 515.

Anhand der zeitlichen Abfolge der so gekennzeichneten 515 Merkmalsvektoren werden anschließend Übergangspunkte identifiziert 520, an denen einen empirisch vorgebbaren Schwellenwert 525 überschreitende Änderungen auftreten. Zur Identifizierung 520 der Übergangspunkte wird in dem vorliegenden Ausführungsbeispiel ein multivarianter Algorithmus zur Wechselpunkterfassung angewendet.

Anhand der so identifizierten 520 Übergangspunkte werden ähnliche, zeitlich aufeinander folgende Fenster zu Segmenten unterschiedlicher Länge gruppiert 530. Dabei entspricht jedes Segment einer anderen Art von fahrbetriebsbedingter körperlicher Belastung für die Fahrzeuginsassen. Für Segmente, die als relativ abrupte Ereignisse klassifiziert werden können, wird in dem vorliegenden Ausführungsbeispiel eine zusätzliche Wavelet-Analyse an genannten Fenstern durchgeführt 535, um auch den Ort und die Richtung der jeweiligen Einwirkungen auf das Fahrzeug und damit möglichen Auswirkungen auf das Wohlbefinden bzw. den Gesundheitszustand des jeweiligen Insassen ermitteln zu können.

Bei den Auswirkungen auf das Wohlbefinden des jeweiligen Insassen treten sowohl die Ursache, d.h. die Erfassung bzw. Ermittlung des Fahrzustandes, als auch die Wirkung, d.h. fahrbetriebsbedingte körperliche Symptome einer möglichen Kinetose, mit einer erheblichen zeitlichen Latenz von bis zu mehreren Minuten auf. Diese Latenzzeit kann, neben den Vitalfunktionen, als zusätzlicher Lernparameter des Auswertealgorithmus herangezogen werden. Der Algorithmus muss dazu in der Lage sein, eine fahrbetriebsbedingte körperliche Belastungserscheinung mit einem in der Vergangenheit stattgefundenen, die Belastungserscheinung kausal bewirkenden Fahrzustand zu verknüpfen.

Die Merkmalsvektoren von Fenstern innerhalb jedes Segments können, wie durch die Strichelung angedeutet, vor der Klassifizierung 530 zusätzlich aggregiert werden 540. Die Klassifizierung 530 erfolgt in dem Ausführungsbeispiel mittels eines genannten "Random Forest"-Klassifizierers, wobei vorliegend zu Vereinfachungszwecken nur der Straßenoberflächentyp sowie die Fahrzeugbeschleunigungen in den drei Achsen klassifiziert werden 530.

Bei dem Random Forest-Klassifizierer wird bekanntermaßen aus mehreren unkorrelierten Entscheidungsbäumen, unter Verwendung eines Zufallsmechanismus', ein Lernprozesses gebildet. Für die Klassifikation trifft dabei jeder Entscheidungsbaum eine Entscheidung, wobei die Klasse mit den meisten Stimmen die endgültige Klassifikation festlegt. Die Parameter und Hyperparameter des Klassifikators können anhand von Trainingsdaten angelernt bzw. trainiert werden, wobei entsprechende Trainingsdaten an einem Teststand oder im realen Straßenverkehr mit jeweiligen Testpersonen erstellt werden können.

Die Einbeziehung der genannten Korrelation zwischen fahrdynamischen Größen und dem subjektiven Feedback seitens des bzw. der Fahrzeuginsassen bei der Auswertung der insgesamt vorliegenden Daten erfolgt in dem vorliegenden Ausführungsbeispiel dadurch, dass die in der beschriebenen Weise klassifizierten fahrdynamischen Daten z.B. mittels der auf dem externen Rechnersystem vorliegenden KI 135 abgeglichen bzw. korreliert werden. Dabei werden bestimmte fahrdynamische Klassen bestimmten Insassenfeedback-Typen zugeordnet. So kann z.B.
- die Fahrdynamikklasse "Geradeausfahrt mit einer Beschleunigung größer als XY" dem einem vegetativen Symptom einer drohenden oder bereits bestehenden Kinetose entsprechenden Feedbacktyp "Müdigkeit" oder
- die Fahrdynamikklasse "Kurvenfahrt durch eine Rechtskurve mit einer Geschwindigkeit größer YZ" (oder entsprechend eine "Kurvenfahrt durch eine Linkskurve") dem einem weiteren vegetativen Symptom bzw. vestibulärem Reiz gemäß einer drohenden Kinetose entsprechenden Feedbacktyp "massive Übelkeit" zugeordnet werden.

Die Insassenfeedback-Typen können während des Fahrbetriebs des Fahrzeugs durch explizite Benutzereingabe(n) seitens wenigstens eines Fahrzeuginsassen und/oder anhand von während des Fahrbetriebs an dem wenigsten einen Fahrzeuginsassen durchgeführten Vitalitätsmessungen, z.B. Messungen der Pulsfrequenz und/oder der Atemfrequenz des Fahrzeuginsassen, erzeugt bzw. bereitgestellt werden.

Die so ermittelten Zuordnungen können danach durch ein im Fahrbetrieb des Fahrzeugs durchgeführtes KI-basiertes Lernen weiter angepasst bzw. verfeinert werden.

Die gesamte in den Figuren 2 bis 4 beispielhaft gezeigte Prozessroutine stellt somit einen während des Fahrbetriebs des autonomen Fahrzeugs fortwährend ablaufenden, geschlossenen Prozess dar, wodurch sichergestellt ist, dass die wenigstens von dem Beschleunigungssensor gelieferten Rohdaten in jeder Fahrbetriebsphase des Fahrzeugs erfasst und an den externen Rechner übermittelt werden. Dadurch ist es möglich, den hier betroffenen, fahrbetriebsbedingten Gesundheitszustand des bzw. der jeweiligen Fahrzeuginsassen lückenlos für die Auswertung an dem externen Rechner zur Verfügung stellen zu können.

## Patentansprüche

1. Verfahren zum Ermitteln eines fahrbetriebsbedingten Gesundheitszustandes wenigstens eines Insassen eines Fahrzeugs, wobei eine sensorische Erfassung wenigstens einer fahrdynamischen Größe im Fahrbetrieb des Fahrzeugs vorgesehen ist, wobei anhand der wenigstens einen sensorisch erfassten fahrdynamischen Größe auf eine mögliche Beeinflussung des fahrbetriebsbedingten Gesundheitszustandes des wenigstens einen Insassen geschlossen wird, und wobei die wenigstens eine sensorisch erfasste fahrdynamische Größe zur Auswertung in Bezug auf eine möglicherweise drohende Beeinflussung des Gesundheitszustandes des wenigstens einen Insassen aus dem Fahrzeug drahtlos an einen externen Rechner (125) übertragen wird, **dadurch gekennzeichnet, dass** die Sensordaten in dem Fahrzeug fortwährend ausgelesen und zwischengespeichert werden, dass innerhalb eines vorgebbaren Zeitfensters erfasste Sensordaten auf das Vorliegen eines für die Ermittlung des fahrbetriebsbedingten Gesundheitszustandes des wenigstens einen Insassen relevantes Fahrereignis hin geprüft werden, wobei das Überschreiten eines empirisch vorgebbaren Schwellenwertes geprüft wird, und dass bei erkanntem Vorliegen eines für den Gesundheitszustand relevanten Ereignisses die zwischengespeicherten Sensordaten des betreffenden Zeitfensters an den externen Rechner (125) übermittelt werden, wobei bei nicht erkanntem Vorliegen eines relevanten Ereignisses in dem betreffenden Zeitfenster Nulldaten an den externen Rechner (125) übermittelt werden.

2. Verfahren nach Anspruch 1, **dadurch gekennzeichnet, dass** der externe Rechner (125) ein lernfähiges Auswertesystem (130) umfasst, welches geeignete Maßnahmen zur wirksamen Verhinderung einer Beeinflussung des Gesundheitszustandes des wenigstens einen Insassen vorschlägt.

3. Verfahren nach einem der vorhergehenden Ansprüche, **dadurch gekennzeichnet, dass** Vitalfunktionen des wenigstens einen Insassen erfasst werden und die so erfassten Daten (105) der Vitalfunktionen mit den sensorisch erfassten fahrdynamischen Größen korreliert werden,
insbesondere, **dadurch gekennzeichnet, dass** bei der Korrelierung der Daten der Vitalfunktionen mit den fahrdynamischen Größen für den wenigstens einen Insassen individuelle medizinische Dispositionsdaten berücksichtigt werden, welche in einem empirisch vorgebbaren Zeitraum ermittelt und/oder verfeinert werden.

4. Verfahren nach einem der vorhergehenden Ansprüche bei einem autonom mit einer Fahrstrategie betriebenen Fahrzeug, **dadurch gekennzeichnet, dass** die Fahrstrategie des Fahrzeugs angepasst wird, um der Beeinflussung des Gesundheitszustandes des wenigstens einen Insassen entgegenzuwirken,
insbesondere **dadurch gekennzeichnet, dass** zum Entgegenwirken der Beeinflussung des Gesundheitszustandes des wenigstens einen Insassen zusätzlich wenigstens eine der folgenden Maßnahmen durchgeführt wird:
- Körperliche Maßnahmen;
- umgebungsbezogene Maßnahmen;
- technisch bezogene Maßnahmen.

5. Verfahren nach einem der vorhergehenden Ansprüche, **dadurch gekennzeichnet, dass** die zur Erfassung der wenigstens einen fahrdynamischen Größe gelieferten Sensordaten anhand der folgenden Prozessschritte ausgewertet werden:
- Fensterung (300) der erfassten Sensordaten in eine empirisch vorgebbare Fensterlänge,
- Merkmalsextraktion (305) zur Bereitstellung charakteristischer Merkmale in den Sensordaten anhand von zu erzeugenden Merkmalsvektoren,
- Segmentierung (310) der erzeugten Fenster in Segmente unterschiedlicher Länge,
- Klassifizierung (315) der Sensordaten anhand der erzeugten Merkmalsvektoren,
insbesondere wobei das Verfahren bei einem autonom mit einer vorgegebenen Fahrstrategie betriebenen Fahrzeug verwendet wird, **dadurch gekennzeichnet, dass** bei der Merkmalsextraktion (305) die charakteristischen Merkmale in den Sensordaten in Bezug auf die Fahrstrategie des Fahrzeugs bereitgestellt werden und/oder dass die Klassifizierung (315) der Sensordaten zur Ermittlung wenigstens einer Anpassung der Fahrstrategie des Fahrzeugs erfolgt.

6. Verfahren nach Anspruch 5, **dadurch gekennzeichnet, dass** bei der Merkmalsextraktion (305) für jedes durch die Fensterung (300) erzeugte Fenster verschiedene charakteristische Merkmale extrahiert werden. und dass die extrahierten charakteristischen Merkmale bei der Klassifizierung (315) als Zeitdomänenmerkmale oder Frequenzdomänenmerkmale zugrunde gelegt werden,
insbesondere, **dadurch gekennzeichnet, dass** anhand von extrahierten charakteristischen Merkmalen jedes erzeugte Fenster durch einen Merkmalsvektor gekennzeichnet wird.

7. Verfahren nach einem der Ansprüche 5 oder 6, **dadurch gekennzeichnet, dass** bei der Segmentierung (310) Übergangspunkte in der Folge von Merkmalsvektoren identifiziert werden, an denen einen empirisch vorgebbaren Schwellenwert überschreitende Änderungen auftreten.

8. Computerprogramm, welches eingerichtet ist, jeden Schritt eines Verfahrens gemäß einem der Ansprüche 1 bis 7 durchzuführen.

9. Steuergerät eines Fahrzeugs, welches eingerichtet ist, durch Aufspielen des Computerprogramms gemäß Anspruch 8 auf einen Mikroprozessor oder Mikrocontroller des Steuergerätes das Verfahren gemäß einem der Ansprüche 1 bis 7 auszuführen.

10. Einrichtung, welche zur Ermittlung eines fahrbetriebsbedingten Gesundheitszustandes wenigstens eines Insassen eines Fahrzeugs gemäß dem Verfahren nach einem der Ansprüche 1 bis 7 eingerichtet ist, wobei eine sensorische Erfassung wenigstens einer fahrdynamischen Größe im Fahrbetrieb des Fahrzeugs vorgesehen ist, **gekennzeichnet durch** eine an dem Fahrzeug angeordnete Verarbeitungs- und Visualisierungseinheit (110), um die erfassten Sensordaten in dem Fahrzeug vorzuverarbeiten und die vorverarbeiteten Sensordaten mittels einer Datenübertragungseinheit (120) drahtlos an einen externen Rechner (125) zu übertragen, welcher ein Auswertesystem (130) umfasst, welches die Sensordaten mittels eines künstlichen neuronalen Netzwerks (135) auswertet und den fahrbetriebsbedingten Gesundheitszustand des wenigstens einen Insassen ermittelt.

11. Einrichtung nach Anspruch 10, **dadurch gekennzeichnet, dass** mittels des künstlichen neuronalen Netzwerks (135) wenigstens eine Maßnahme zum Entgegenwirken einer Beeinflussung des Gesundheitszustandes des wenigstens einen Insassen ermittelt wird.

12. Einrichtung nach Anspruch 10 oder 11, **gekennzeichnet durch** wenigstens zwei Sensoren (100, 105) unterschiedlicher Kategorie, wobei der erste Sensor (100) zur Erfassung von fahrdynamischen Daten des Fahrzeugs und der zweite Sensor (105) zur Erfassung von Vitalfunktionen des wenigstens einen Insassen dienen.

13. Einrichtung nach einem der Ansprüche 10 bis 12, **dadurch gekennzeichnet, dass** wenigstens ein Sensor (100) zur sensorischen Erfassung der wenigstens einen fahrdynamischen Größe durch einen Bewegungssensor gebildet ist, der dreiachsig die Beschleunigung sowie die Winkeländerung insbesondere in einem für das Entstehen einer Kinetose relevanten, niederfrequenten Bereich von ca. 0,1 bis 0,8 Hz erfasst, insbesondere, **dadurch gekennzeichnet, dass** der wenigstens eine Sensor (100) zusätzlich durch einen Temperatursensor und/oder einen Luftfeuchtigkeitssensor gebildet ist.

14. Einrichtung nach einem der Ansprüche 10 bis 13, **dadurch gekennzeichnet, dass** wenigstens ein Sensor (100) zur sensorischen Erfassung der wenigstens einen fahrdynamischen Größe in dem Fahrzeug bzw. der Fahrzeugelektronik integriert angeordnet ist oder mittels eines mobilen Endgerätes bereitgestellt wird,
insbesondere, **dadurch gekennzeichnet, dass** das mobile Endgerät eine mobile Datenverbindung zum Internet bereitstellt, um die von dem mobilen Endgerät erfassten Sensordaten an den externen Rechner (125) zu übermitteln und von dem Auswertesystem (130) des externen Rechner (125) Auswerteergebnisse zu erhalten, und dass das mobile Endgerät eine Benutzerschnittstelle aufweist, um dem wenigstens einen Insassen einen kritischen Bewegungszustand des Fahrzeugs mitzuteilen und von dem Insassen eine Rückmeldung über seinen Gesundheitszustand einzuholen.

15. Einrichtung nach einem der Ansprüche 10 bis 14, **gekennzeichnet durch** ein Sensormodul (100), welches kontinuierlich Sensordaten mit einer vorgebbaren Abtastrate bereitstellt, einen Mikrocontroller (110) zur Reduzierung der Datenmenge der an den externen Rechner (125) zu übermittelnden Sensordaten, einen Datenspeicher (240) zur Zwischenspeicherung der reduzierten Sensordaten, wobei der Mikrocontroller (110) die zwischengespeicherten (240) Sensordaten in digitale Datenpakete aufteilt, welche mittels der Datenübertragungseinheit (120) drahtlos an den externen Rechner (125) übermittelbar sind.

## Claims

1. Method for determining a driving operation-related state of health of at least one passenger of a vehicle, wherein a sensor-based detection of at least one driving dynamic variable is provided during driving operation of the vehicle, wherein a possible influence on the driving operation-related state of health of the at least one passenger is inferred on the basis of the at least one sensor-detected driving dynamic variable, and wherein the at least one sensor-detected dynamic driving variable is transmitted wirelessly from the vehicle to an external computer (125) for evaluation with regard to a possible imminent influence on the state of health of the at least onepassenger, **characterised in that** the sensor data in the vehicle is continuously read out and temporarily stored, **in that** sensor data recorded within a predeterminable time window is checked for the presence of a driving event which is relevant for determining the state of health of the at least one passenger as a result of driving, the exceeding of an empirically predeterminable threshold value being checked, and **in that**, if an event relevant to the state of health is detected, the temporarily stored sensor data of the relevant time window is transmitted to the external computer (125), zero data being transmitted to the external computer (125) if a relevant event is not detected in the relevant time window.

2. Method according to claim 1, **characterised in that** the external computer (125) comprises an adaptive evaluation system (130) which suggests suitable measures for effectively preventing an influence on the state of health of the at least one passenger.

3. Method according to one of the preceding claims, **characterised in that** vital functions of the at least one passenger are recorded and the data (105) of the vital functions recorded in this way is correlated with the driving dynamics variables detected by the sensors,
in particular, **characterised in that**, when correlating the data of the vital functions with the dynamic driving variables for the at least one passenger, individual medical disposition data is taken into account, which is determined and/or refined in an empirically predeterminable period of time.

4. Method according to one of the preceding claims in the vehicle operated autonomously with a driving strategy, **characterised in that** the driving strategy of the vehicle is adapted in order to counteract the influence on the state of health of the at least one passenger, in particular **characterised in that**, in order to counteract the influence on the state of health of the at least one passenger, at least one of the following measures is additionally carried out:
- Physical measures;
- environmental measures;
- technical measures.

5. Method according to one of the preceding claims, **characterised in that** the sensor data supplied for recording the at least one dynamic driving variable is evaluated using the following process steps:
- Windowing (300) of the detected sensor data into an empirically predeterminable window length,
- Feature extraction (305) for providing characteristic features in the sensor data on the basis of feature vectors to be generated,
- Segmentation (310) of the generated windows into segments of different lengths,
- Classification (315) of the sensor data using the generated feature vectors,
in particular wherein the method is used in the vehicle operated autonomously with a predetermined driving strategy, **characterised in that** in the feature extraction (305) the characteristic features in the sensor data are provided in relation to the driving strategy of the vehicle and/or **in that** the classification (315) of the sensor data is carried out to determine at least one adaptation of the driving strategy of the vehicle.

6. Method according to claim 5, **characterised in that** in the feature extraction (305) different characteristic features are extracted for each window generated by the windowing (300), and **in that** the extracted characteristic features are used as the basis for the classification (315) as time domain features or frequency domain features, in particular **characterised in that** each generated window is identified by a feature vector on the basis of extracted characteristic features.

7. Method according to one of claims 5 or 6, **characterised in that**, during segmentation (310), transition points are identified in the sequence of feature vectors at which changes exceeding an empirically predeterminable threshold value occur.

8. Computer program adapted to perform each step of a method according to any one of claims 1 to 7.

9. Control unit of a vehicle which is adapted to carry out the method according to one of claims 1 to 7 by loading the computer program according to claim 8 onto a microprocessor or microcontroller of the control unit.

10. Device, which is set up to determine a driving operation-related state of health of at least one passenger of a vehicle according to the method according to one of claims 1 to 7, wherein a sensor-based detection of at least one driving dynamic variable is provided during driving operation of the vehicle, **characterised by** a processing and visualisation unit (110) arranged on the vehicle, in order to preprocess the detected sensor data in the vehicle and to transmit the preprocessed sensor data wirelessly by means of a data transmission unit (120) to an external computer (125), which comprises an evaluation system (130), which evaluates the sensor data by means of an artificial neural network (135) and determines the driving-related state of health of the at least one passenger.

11. Device according to claim 10, **characterised in that** at least one measure for counteracting an influence on the state of health of the at least one passenger is determined by means of the artificial neural network (135).

12. Device according to claim 10 or 11, **characterised by** at least two sensors (100, 105) of different categories, wherein the first sensor (100) is used to detect driving dynamics data of the vehicle and the second sensor (105) is used to detect vital functions of the at least one passenger.

13. Device according to one of claims 10 to 12, **characterised in that** at least one sensor (100) for sensor-based detection of the at least one dynamic driving variable is formed by a movement sensor, which detects the acceleration and the angular change in three axes, in particular in a low-frequency range of approximately 0.1 to 0.8 Hz relevant for the occurrence of kinetosis, in particular **characterised in that** the at least one sensor (100) is additionally formed by a temperature sensor and/or a humidity sensor.

14. Device according to one of claims 10 to 13, **characterised in that** at least one sensor (100) for sensor-based detection of the at least one driving dynamic variable is arranged integrated in the vehicle or the vehicle electronics or is provided by means of a mobile terminal device, in particular the vehicle electronics or is provided by means of a mobile terminal device, **characterised in** particular **in that** the mobile terminal device provides a mobile data connection to the Internet in order to transmit the sensor data recorded by the mobile terminal device to the external computer (125) and to obtain evaluation results from the evaluation system (130) of the external computer (125), and **in that** the mobile terminal device has a user interface in order to inform the at least one passenger of a critical movement state of the vehicle and to obtain feedback from the passenger about his state of health.

15. Device according to one of claims 10 to 14, **characterised by** a sensor module (100) which continuously provides sensor data at a predeterminable sampling rate, a microcontroller (110) for reducing the amount of data of the sensor data to be transmitted to the external computer (125), a data memory (240) for temporarily storing the reduced sensor data, wherein the microcontroller (110) divides the temporarily stored (240) sensor data into digital data packets which can be transmitted wirelessly to the external computer (125) by means of the data transmission unit (120).

## Revendications

1. Procédé pour déterminer un état de santé dépendant du mode de conduite d'au moins un occupant d'un véhicule, dans lequel il est prévu une détection par capteur d'au moins une grandeur dynamique de conduite en mode de conduite du véhicule, 'au moins une grandeur dynamique de conduite détectée par le capteur permettant de conclure à une influence possible de l'état de santé dépendant du mode de conduite de l'au moins un occupant, et l'au moins une grandeur dynamique de conduite détectée par le capteur étant transmise sans fil du véhicule à un ordinateur externe (125) en vue de l'analyse en rapport avec une influence éventuellement imminente de l'état de santé de l'au moins un occupant, **caractérisé en ce que** données de capteur sont lues en continu et stockées temporairement dans le véhicule, **en ce que** des données de capteur détectées dans une fenêtre temporelle prédéfinissable sont contrôlées en vue d'établir la présence d'un événement de conduite pertinent pour déterminer l'état de santé dépendant du mode de conduite de l'au moins un occupant, le dépassement d'une valeur seuil prédéfinissable de manière empirique étant contrôlé, et **en ce que** la présence d'un événement pertinent pour l'état de santé est détectée, les données de capteur stockées temporairement dans la fenêtre temporelle concernée sont transmises à l'ordinateur externe (125), et si aucun événement pertinent n'est détecté dans la fenêtre temporelle concernée, des données nulles sont transmises à l'ordinateur externe (125).

2. Procédé selon la revendication 1, **caractérisé en ce que** l'ordinateur externe (125) comprend un système d'évaluation (130) capable d'apprendre, qui propose des mesures appropriées pour empêcher efficacement une influence sur l'état de santé de l'au moins un occupant.

3. Procédé selon l'une des revendications précédentes, **caractérisé en ce que** des fonctions vitales de l'au moins un occupant sont détectées et les données (105) ainsi détectées des fonctions vitales sont corrélées avec les grandeurs dynamiques de conduite détectées par capteur, caractérisé en particulier **en ce que** lors de la corrélation sont prises en compte des données des fonctions vitales avec les grandeurs dynamiques de conduite pour l'au moins un occupant, des données de disposition médicale individuelles qui sont déterminées et/ou affinées dans un intervalle de temps pouvant être prédéfini empiriquement.

4. Procédé selon l'une des revendications précédentes pour un véhicule fonctionnant de manière autonome avec une stratégie de conduite, **caractérisé en ce que** la stratégie de conduite du véhicule est adaptée pour s'opposer à l'influence sur l'état de santé de l'au moins un occupant, en particulier **caractérisé en ce que** pour s'opposer à l'influence sur l'état de santé de l'au moins un occupant, au moins l'une des mesures suivantes est également effectuée :
- Mesures physiques ;
- Mesures liées à l'environnement ;
- Mesures liées à la technique.

5. Procédé selon l'une des revendications précédentes, **caractérisé en ce que** les données de capteur fournies pour la saisie de l'au moins une grandeur dynamique de conduite sont évaluées à l'aide des étapes de processus suivantes :
- Fenêtrage (300) des données de capteur détectées en une longueur de fenêtre prédéfinissable empiriquement,
- Extraction de caractéristiques (305) pour fournir des caractéristiques dans les données de capteur à l'aide de vecteurs de caractéristiques à générer,
- Segmentation (310) des fenêtres générées en segments de différentes longueurs,
- Classification (315) des données de capteur sur la base des vecteurs de caractéristiques générés, en particulier le procédé étant utilisé pour un véhicule fonctionnant de manière autonome avec une stratégie de conduite prédéfinie, **caractérisé en ce que** lors de l'extraction de caractéristiques (305), les caractéristiques dans les données de capteur sont mises à disposition en relation avec la stratégie de conduite du véhicule et/ou **en ce que** la classification (315) des données de capteur est effectuée pour déterminer au moins une adaptation de la stratégie de conduite du véhicule.

6. Procédé selon la revendication 5, **caractérisé en ce que**, lors de l'extraction de caractéristiques (305), différentes caractéristiques sont extraites pour chaque fenêtre générée par le fenêtrage (300), et **en ce que** les caractéristiques extraites sont utilisées comme base pour la classification (315) en tant que caractéristiques du domaine temporel ou caractéristiques du domaine fréquentiel, en particulier, **caractérisé en ce que**, à l'aide de caractéristiques extraites, chaque fenêtre générée est **caractérisée par** un vecteur de caractéristiques.

7. Procédé selon l'une des revendications 5 ou 6, **caractérisé en ce que**, lors de la segmentation (310), des points de transition sont identifiés dans la séquence de vecteurs de caractéristiques, au niveau desquels apparaissent des modifications dépassant une valeur seuil pouvant être prédéfinie empiriquement.

8. Programme informatique conçu pour exécuter chaque étape d'un procédé selon l'une des revendications 1 à 7.

9. Appareil de commande d'un véhicule, qui est conçu pour exécuter le procédé selon l'une des revendications 1 à 7 par chargement du programme informatique selon la revendication 8 sur un microprocesseur ou un microcontrôleur de l'appareil de commande.

10. Dispositif conçu pour déterminer un état de santé dépendant du mode de conduite d'au moins un occupant d'un véhicule, conformément au procédé selon l'une des revendications 1 à 7, une saisie par capteur d'au moins une grandeur dynamique de conduite étant prévue en mode de conduite du véhicule, **caractérisé par** une unité de traitement et de visualisation (110) disposée sur le véhicule, pour prétraiter les données de capteur détectées dans le véhicule et pour transmettre sans fil les données de capteur prétraitées au moyen d'une unité de transmission de données (120) à un ordinateur externe (125) qui comprend un système d'évaluation (130) qui évalue les données de capteur au moyen d'un réseau neuronal artificiel (135) et qui détermine l'état de santé dépendant du mode de conduite de l'au moins un occupant.

11. Dispositif selon la revendication 10, **caractérisé en ce qu'**au moyen du réseau neuronal artificiel (135), au moins une mesure est déterminée pour contrer une influence sur l'état de santé de l'au moins un occupant.

12. Dispositif selon la revendication 10 ou 11, **caractérisé par** au moins deux capteurs (100, 105) de catégories différentes, le premier capteur (100) servant à l'acquisition de données dynamiques de conduite du véhicule et le deuxième capteur (105) à l'acquisition de fonctions vitales d'au moins un occupant.

13. Dispositif selon l'une des revendications 10 à 12, **caractérisé en ce qu'**au moins un capteur (100) pour la détection sensorielle de l'au moins une grandeur dynamique de conduite est formé par un capteur de mouvement qui détecte sur trois axes l'accélération ainsi que la variation angulaire, en particulier dans une plage basse fréquence d'environ 0,1 à 0,8 Hz, pertinente pour l'apparition d'une cinétose, en particulier, **caractérisé en ce que** l'au moins un capteur (100) est formé en outre par un capteur de température et/ou un capteur d'humidité de l'air.

14. Dispositif selon l'une des revendications 10 à 13, **caractérisé en ce qu'**au moins un capteur (100) pour la saisie sensorielle de l'au moins une grandeur dynamique de conduite est intégré au véhicule ou à l'appareil électronique du système électronique du véhicule ou est mis à disposition au moyen d'un terminal mobile, **caractérisé en ce que** le terminal mobile met à disposition une connexion de données mobile à Internet pour transmettre les données de capteur détectées par le terminal mobile à l'ordinateur externe (125) et pour obtenir des résultats d'évaluation du système d'évaluation (130) de l'ordinateur externe (125), et **en ce que** le terminal mobile présente une interface utilisateur pour communiquer à l'au moins un occupant un état de mouvement critique du véhicule et pour obtenir de l'occupant un retour sur son état de santé.

15. Dispositif selon l'une des revendications 10 à 14, **caractérisé par** un module de capteur (100) qui met à disposition en continu des données de capteur avec un taux d'échantillonnage pouvant être prédéfini, un microcontrôleur (110) pour réduire la quantité de données des données de capteur à transmettre à l'ordinateur externe (125), une mémoire de données (240) pour le stockage intermédiaire des données de capteur réduites, le microcontrôleur (110) divisant les données de capteur (240) stockées temporairement en paquets de données numériques qui peuvent être transmis sans fil à l'ordinateur externe (125) au moyen de l'unité de transmission de données (120).
